# EUROPEAN PATENT SPECIFICATION

(11) **EP 0 556 281 B1**
(45) Date of publication and mention of the grant of the patent: **06.02.2002**
(21) Application number: 91920633.4
(22) Date of filing: 31.10.1991
(51) Int. Cl.: G01N 33/569, C12Q 1/04

(54) **A DIAGNOSTIC ASSAY FOR LYME DISEASE**
DIAGNOSTISCHER TEST FÜR LYME-KRANKHEIT
TEST DIAGNOSTIC DE LA MALADIE DE LYME

(30) Priority: 31.10.1990 US 605798
(43) Date of publication of application: 25.08.1993
(73) Proprietor: GUNDERSEN MEDICAL FOUNDATION, LaCrosse, WI 54601 (US)
(72) Inventor: CALLISTER, Steven, M., Onalaska, WI 54650 (US); SCHELL, Ronald, F., Madison, WI 53706 (US)
(74) Representative: Brown, John David
(86) International application number: US9108047
(87) International publication number: WO9208135

(56) References cited:
- JOURNAL OF CLINICAL MICROBIOLOGY vol. 29, no. 9, 1 September 1991, WASHINGTON DC USA pages 1773 - 1776; S.M. CALLISTER ET AL.: 'Lyme disease assay which detects killed Borelia burgdoreri.' See whole article.
- JOURNAL OF CLINICAL MICROBIOLOGY vol. 27, no. 4, 1 April 1989, WASHINGTON DC USA pages 773 - 774; S.M. CALLISTER ET AL.: 'Efficacy of the urinary bladder for isolation of Borrelia burgdorferi from naturally infected wild Peromyscus leucopus.' cited in the application See whole article.
- JOURNAL OF CLINICAL MICROBIOLOGY vol. 26, no. 3, 1 March 1990, WASHINGTON DC USA pages 473 - 479; M. KARLSSON ET AL.: 'Cultivation and characterization of spirochetes from cerebrospinal fluid of patients with lyme borreliosis.' See whole article.

## Description

### FIELD OF THE INVENTION

The present invention relates to clinical methods of detecting and evaluating infection of a patient with Borrelia burgdorferi, the organism responsible for Lyme disease. The method of the present invention can also be used to evaluate the immune status of a patient (e.g., to detect the presence of antibodies after immunization) and success of therapy (e.g., a decrease in antibody titer).

### BACKGROUND OF THE INVENTION

Lyme disease, named after the site of an epidemic of oligoarticular arthritis [Steere et al.. Arthritis Rheum. 20: 7-17 (1977)], is a multisystem infection often accompanied by an expanding skin lesion (erythema migrans) and concomitant or subsequent development or arthritic, cardiac, or neurologic complications [Reik et al., Medicine 58: 281-294 (1979); Steere et. al., Ann. Intern. Med 93 (1) 8-16 (1980); Steere et al.., Ann. Intern. Med. 86: 685-698 (1977); Steere et al., Ann. Intern. Med. 107: 725-731 (1987)]. Subsequent epidemiological studies have identified the deer tick, Ixodes dammini, as the primary vector of Lyme disease in North America [Burgdorfer et al., Science 216 1317-1319 (1982)] and a spirochete, Borrelia burgdorferi (hereinafter "B. burgdorferi"), as the causative agent of Lyme disease [Anderson et al., Am. J. Trop. Med. 32: 818-824 (1983); Steere et al., N. Engl. J. Med. 308: 733-740 (1983)]. Lyme disease is now the most common tick-borne illness recognized in the United States [Habicht et. al., Sci. Am. 257: 78-83 (1987)].

Since Lyme disease may be more successfully treated if diagnosed early, an effective clinical assay for the detection of exposure to or infection with B. burgdorferi has been sought. Several prior assays, which rely on determination of the titer of antibodies to B. burgdorferi in patient sera, have used fluorescent-labelled anti-IgG antibodies [e.g., Magnarelli et. al., J. Clin. Microbiol. 20: 181-184 (1984); Russell et al., J. Infect. Dis. 149: 465-470 (1984); Steere et al., N. Engl. J. Med. 308: 733-740 (1983); Stiernstedt et al., J. Clin. Microbiol. 21: 819-825 (1985); Pennell et al., J. Clin. Microbiol. 25: 2218-2220 (1987)] and enzyme-labelled anti-IgG antibodies [e.g., Craft et al., J. Infect. Dis. 149: 789-795 (1984); Magnarelli et al., J. Clin. Microbiol. 20: 181-184 (1984); Russell et al., J. Infect. Dis. 149: 465-470 (1984); Stiernstedt et al., J. Clin. Microbiol. 21: 819-825 (1985); Berardi et al., J. Infect. Dis. 158: 754-760 (1988)] to detect anti-B. burgdorferi antibodies which had previously been immobilized on a solid phase by binding to B. burgdorferi antigen. Unfortunately, these assays are of limited clinical use, since these antibody titers may not accurately indicate when the patient was exposed to B. burgdorferi. Furthermore, in many cases antibodies are not detected in patient sera by such assays until major symptoms of Lyme disease begin to appear making effective treatment more difficult if not impossible. In addition, these assays fail to define the immune status of the host or to predict the response of the host to infection or re-infection. It would, therefore, be desirable to provide an assay for exposure to or infection with B. burgdorferi which is capable of more accurate and more contemporaneous (i.e., closer to the date of exposure or infection) detection and which is capable of defining the immune status of a patient.

### DESCRIPTION OF THE FIGURES

Figure 1 is a table summarizing the reduction in the number of viable B. burgdorferi organisms in samples treated in Example 2 below.

Figure 2 is a table summarizing the reduction in the number of viable B. burgdorferi organisms in samples treated in Example 3 below.

Figure 3 is a graph relating to the continued growth (as described in Example 4) of samples after treatment in accordance with the methods of the present invention.

Figure 4 is a graph relating to the effect of organism age on the method of the present invention.

Figure 5 is a graph relating to the effect of incubation time on the method of the present invention.

Figure 6 is a graph relating to the effect of patient serum dilution on the method of the present invention.

Figure 7 is a graph comparing the immobilizing/lytic activity of normal serum and BSK medium.

Figures 8-16 are graphs and tables summarizing data collected in Examples 11-21.

### SUMMARY OF THE INVENTION

In accordance with the present invention, an in vitro method of performing an assay to determine whether a patient has been exposed to Borrelia burgdorferi (i.e., infected by, immunized or vacoinated with respect to or otherwise exposed to Borrelia burgdorferi) is disclosed which comprises collecting serum from the patient; preparing a sample mixture comprising a portion of the patient's serum and an inoculum of viable Borrelia burgdorferi organisms; incubating the sample mixture; determining the number of viable organisms remaining in the sample mixture after incubation; and comparing the number with the quantity of viable organisms remaining in a control. The patient can be either human or animal. The patient serum can be heat inactivated before conducting the assay such that immobilization or cell lysis is observed. In some instances, a source of complement can be added to the sample mixture. Serum also may be treated by ion exchange or other absorbing materials (e.g., cellulose phosphate) to remove anti-microbial agents if present.

By using the teachings of the present invention an assay can be performed "to determine whether a patient has been exposed to or infected by B. burgdorferi" regardless of the source of such exposure. For example, exposure can come in the form of infection by tick bite or by injection of B. burgdorferi or antigen in the form of a Lyme disease vaccine. In either event, the present invention can be employed to detect exposure or to determine the immune status of the patient.

Although B. burgdorferi, strain 297 is preferred for conducting the assay of the present invention, many strains can be used (e.g., strain B31). The methods of the present invention have been performed using numerous other strains including IS-17, Chicago, European and S-1-10 isolates. A strain which is common to the area in which the patient may have been exposed to B. burgdorferi may be preferred over other strains, if available. Preferably, the organisms used in the assay are "properly aged". "Properly aged" organisms are those that are at least about 72-74-hours old or in log phase growth, or those which have been subjected to a process which provides a similar effect in the sensitivity of the assay as aging (e.g., lyophilization of the organisms). A deposit of B. burgdorferi, strain 297 has been deposited with ATCC in accordance with the Budapest treaty on April 21, 1989, as accession number 53899. B. burgdorferi, strain B31 is also available from ATCC as accession number 35210. Strains of B. burgdorferi can also be isolated according to the methods of Callister et al., J. Clin. Microbiol. 27: 773-774 (1989), which is incorporated herein in its entirety as if fully set forth. Although an inoculum of 10⁴-10⁵ organisms is preferred for practicing the method of the present invention, other concentrations of organism can be used.

Incubation can be performed in any way known to the art which will promote growth of the organisms. Although longer or shorter incubation times can be used, preferably the samples are incubated at 32 C for 30 minutes to 18 hours.

Preferably, the control is prepared by forming a control mixture of normal serum and an inoculum of viable Borrelia burgdorferi organisms and incubating the control mixture with the sample mixture.

Samples and controls are observed, preferably under a microscope, and the number of viable organisms are counted and compared to controls. Other means for counting viable organisms can also be used for practicing the present invention. For example, the organisms used to inoculate each sample could be labelled with a radioisotope, enzyme, fluorescent moiety, chemiluminescent moiety or other label such that the number or organisms can be determined by measuring the presence of the labeling material. Flow cytometry (employing known dyes such as propidium iodide) could be used to differentiate live and dead organisms. Samples could also be filtered after treatment and the remaining organisms on the filter could be determined. Catabolic or metabolic products or processes of the organisms could be measured to either determine the number of remaining organisms or the number of organisms which have been lysed and have released cell contents, or that differentiate viable and non-viable organisms. For example, the uptake of ³H-adenine could be monitored. Differences in the synthesis of DNA or RNA by patient and control samples could also be observed. Other means for quantitating the number of organisms remaining after treatment in accordance with the methods of the present invention or the number of organisms lysed, killed or immobilized by such treatment will be apparent to those skilled in the art and can be utilized in practicing the present invention.

"Viable organisms" are those organisms (a) which have not been lysed or have not disappeared, (b) which have not been immobilized or partially immobilized, and/or (c) which do not exhibit loss of refractivity. The number of viable organisms is counted because certain embodiments of the present invention may result in agglutination or complete lysis and disappearance of cells which, as a result, become unobservable.

As an article of manufacture, an assay kit is also disclosed which is useful for determining whether a patient has been exposed to or infected by B. burgdorferi (or to determine the immune status of a patient). The kit contains reagents nacessary to practice the assay method disclosed herein. In its broadest form, the kit comprises an inoculum of viable B. burgdorferi organisms, an aliquot of immune or normal serum and an aliquot of BSK medium. Other reagents, tubes, and other materials can also be included in the kits, such as complement or labelled or labelling moieties for use in detecting viable organisms (e.g., ³H-adenine or propidium iodide). The components of the kit can be viable and packaged by standard means applied in the diagnostic industry.

A method of conferring passive immunity upon a Lyme-susceptible (i.e., capable of being infected by B. burgdorferi) individual is also disclosed. The method comprises administering to the individual an amount of Borrelia burgdorferi positive (i.e., has been determined to have been taken from an individual who had been exposed to or infected with B. burgdorferi) serum sufficient to provide passive immunity, wherein the serum is determined to be positive according to the assay methods of the present invention.

### DETAILED DESCRIPTION OF THE INVENTION

Throughout the examples presented below, reference is made to Barbour-Stoenner-Kelly ("BSK") medium. BSK medium was prepared in accordance with Barbour, Yale J. Biol. Med 57: 71-75 (1984), which is incorporated herein in its entirety by reference. After formulation, the BSK medium was subjected to a quality control scheme to ensure suitability for use. To check the quality of each batch of BSK medium, B. burgdorferi cultures were prepared which contained approximately 10⁸, 10⁷, 10⁶, 10⁵, 10⁴, 10³, 10², 10 and 1 organism(s) in 6ml BSk medium. The cultures were then incubated at 32C for 3 weeks. After incubation, the cultures were checked to see which culture tubes had shown growth. Preferably, the batch of BSK medium which grew morphologically normal B. burgdorferi from the lowest starting inoculum was used. Although BSK medium which does not meet this criterion can be used in practicing the present invention, it is preferred that the BSK medium pass these quality control measures. It is also preferred that the albumin used in formulating the BSK medium is fresh and of high quality (Callister et al., J. Clin. Microbiol. 28: 363-365 (1990)).

### EXAMPLE 1

Case defined patient sera to be evaluated for exposure to B. burgdorferi were collected. A normal serum pool was made from 10 sera from Wisconsin (which had been tested as negative by immunofluorescent assay (IFA)] patients which had no known exposure to B. burgdorferi. The patient serum and normal serum were diluted 1:5 with BSK medium. The diluted sera were then filter sterilized and heat inactivated at 56C for 45 minutes. sera were freed of anti-microbiols by treatment with IONAC-C249 commercially available from Sybron Corp., New Jersey.

Individual samples were prepared by adding 100ul of heat-inactivated patient serum or normal serum control to 100ul of BSK medium containing approximately 10⁵ B. burgdorferi organisms. No source of complement was added. The samples were then incubated for 6 hours at 32C. After incubation, the incubated samples were vortexed for one minute.

3x 10ul of each sample and control were placed onto slides for counting. Viable organisms and organisms which were not immobilized were counted and the sample and control values were compared.

It was observed that serum from patient's who had been exposed to B. burgdorferi exhibited a significant decrease in the number of organisms which were viable and not immobilized as compared to the original inoculum. Some organisms were agglutinated or lysed and completely "disappeared." In contrast, the normal serum control showed no significant decrease. As a result, an observed decrease in the number of organisms from the number in the original inoculum is an indication of immune status or exposure to or infection with B. burgdorferi.

### EXAMPLE 2

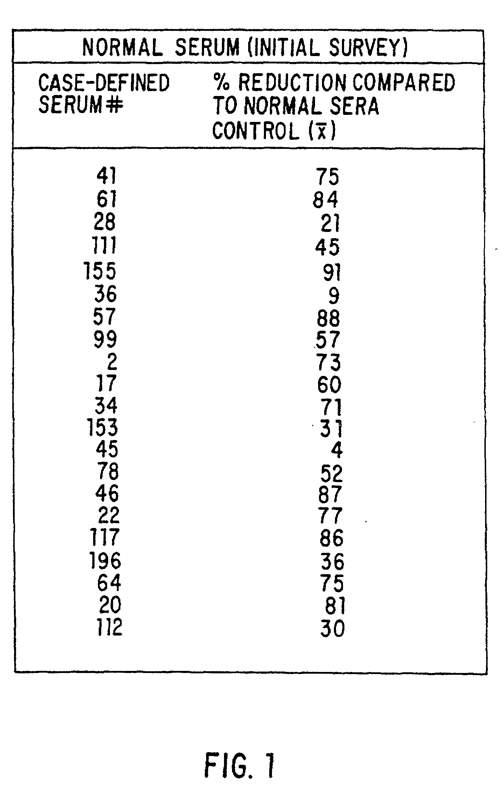

21 frozen samples of patient sera from 1985, which had previously been determined, by fluorescence immunoassay (IFA), as positive for B. burgdorferi infection were thawed and analyzed in accordance with the procedure described above in Example 1, except that the samples were incubated for 6 hours. Before analysis, all samples were filter sterilized (0.22u filter) and/or treated to remove antibiotics. Figure 1 summarizes the percent reduction in the number of B. burgdorferi organisms after treatment in accordance with the present invention. 19 (91%) of the samples tested positive to some degree (i.e., showed a reduction in organism number) in the assay of the present invention. No normal serum controls showed any significant decrease in organism number. The assay of the present invention detected that 90% of the previous case defined samples were, in fact, positive for B. burgdorferi exposure or infection.

### EXAMPLE 3

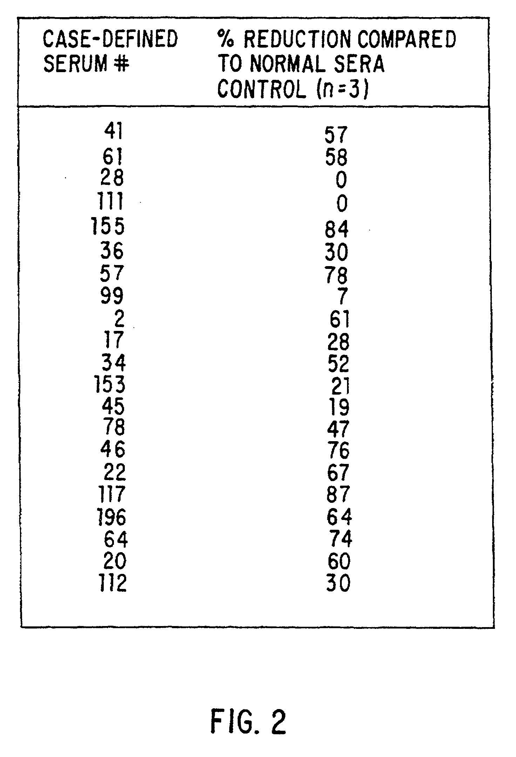

The same 21 samples were again tested as described in Example 2, with the exception that organisms aged for 72 hours were added to each sample and the samples were incubated for 18 hours. Figure 2 summarizes the results of these tests. Again, 19 of 21 samples tested positive in the assay of the present invention. However, the use of aged organisms and a longer incubation time decreased the overall variability of the assay.

### EXAMPLE 4

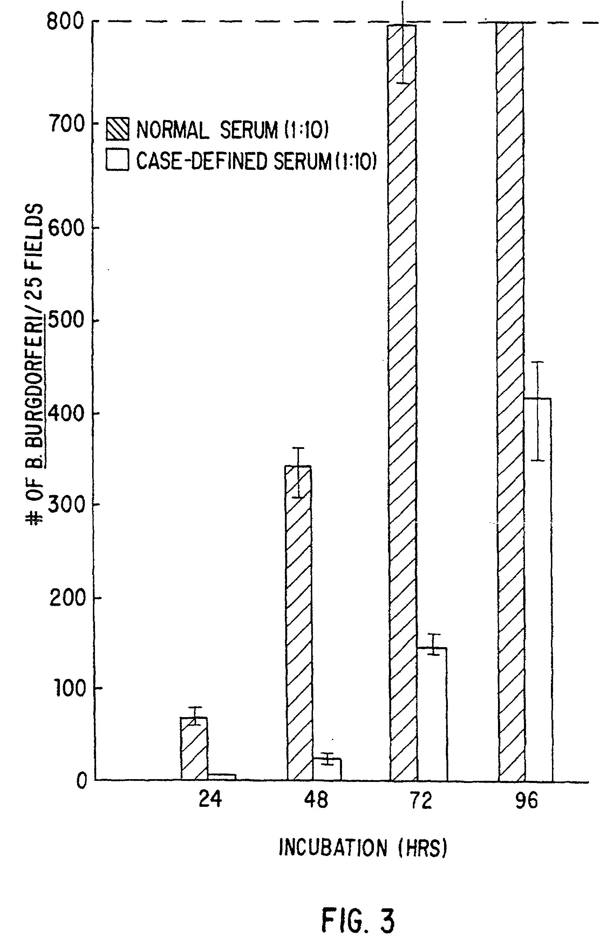

To confirm that the number of remaining viable B. burgdorferi was actually decreased by the method of the present invention, 6ml of fresh BSK medium was added to one patient sample (number 57 in Figures 1 and 2) and a normal serum sample from Example 2. The samples were then reincubated at 32C. The number of organisms in each sample was counted 24, 48, 72, and 96 hours after addition of the fresh BSK medium. The count results are summarized in Figure 3. At each time interval, the normal serum control was observed to contain significantly more organisms than the patient sample, thus confirming that the number of viable organisms in the patient sample had been significantly reduced.

### EXAMPLE 5

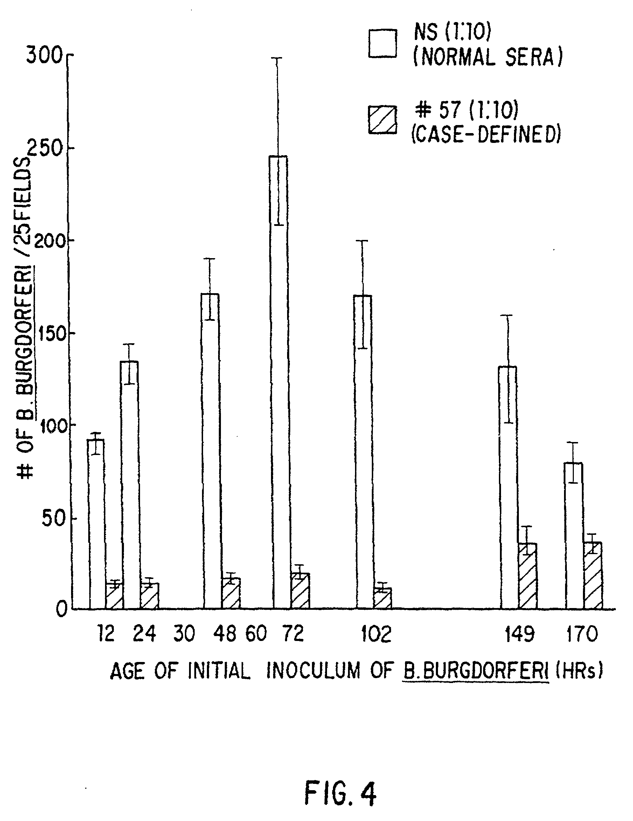

The effect of the age of B. burgdorferi organisms employed in the method of the present invention was examined. Samples were prepared as described above in Example 2, except that organisms were aged for 12, 24, 51, 74, 102, 149 and 170 hours before inoculating separate samples. Test results are summarized in Figure 4. All samples exhibited a significant reduction in the number of viable organisms, although 74-hours organisms appeared to maximize the reduction observed. The results demonstrate that properly aged organisms, or organisms subjected to processes that achieve the same effect (e.g., lyophilization), change the sensitivity of the assay.

### EXAMPLE 6

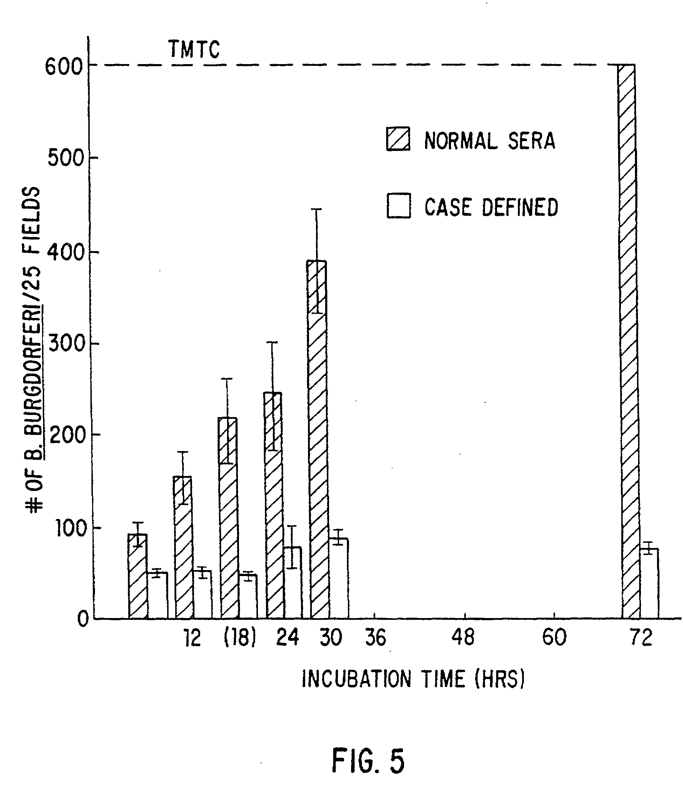

The effect of different incubation times was examined. Samples were prepared as described above in Example 2, except that the assay was performed (counted) after 6, 12, 18, 24, 30 and 72 hour incubation intervals. Test results are summarized in Figure 5. All samples exhibited a significant reduction in the number of viable organisms and the magnitude of the reduction (i.e., sensitivity) increased with longer incubation. Although greater reduction is observed with longer incubation times, time constraints may make longer incubation time impractical. Preferably the incubation time is 30 minutes to 18 hours.

### EXAMPLE 7

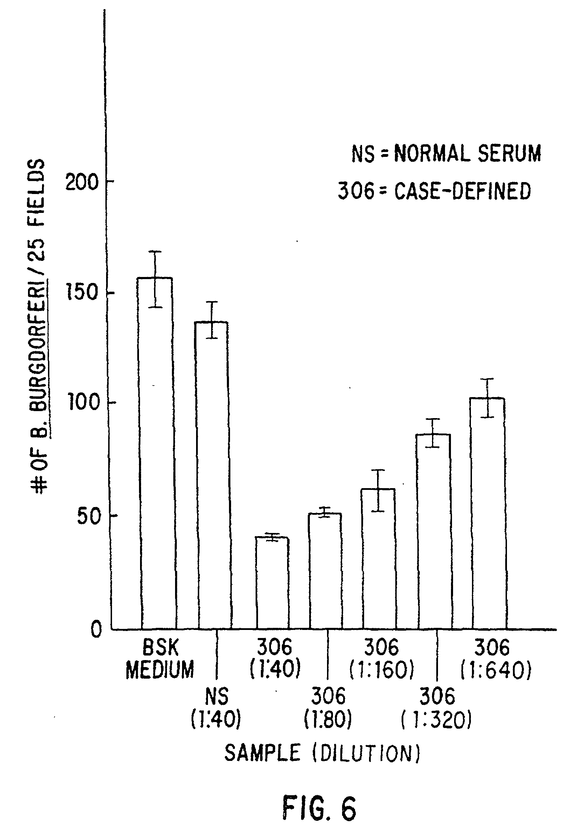

The effect of diluting the patient serum was examined. Samples were prepared as described in Examples 2, except that dilutions of patient sera of 1:40, 1:80, 1:160, 1:320 and 1:640 were prepared. Test results are summarized in Figure 6. Dilution of patient sera significantly decreased the immobilization, agglutination or lytic effect of the method of the present invention.

### EXAMPLE 8

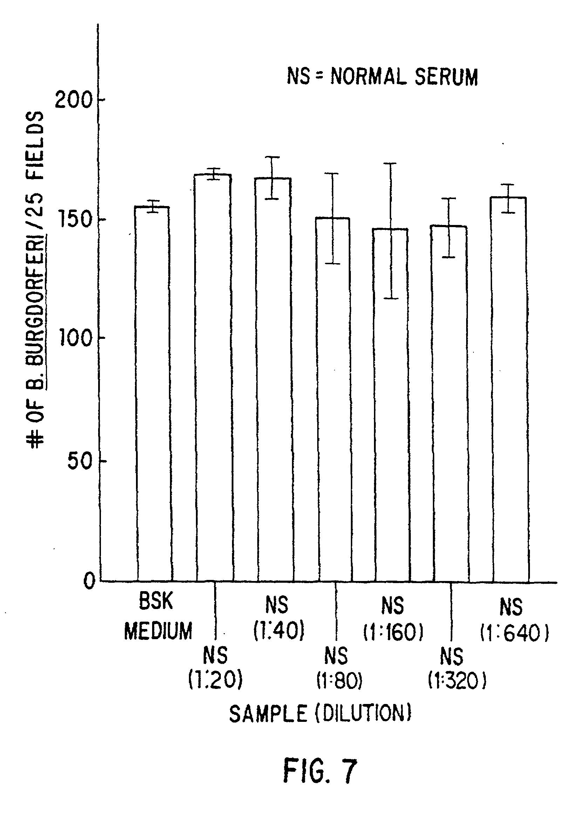

The immobilization, agglutination or lytic effect of a pooled normal serum as compared to BSK medium was examined. Samples of BSK medium and normal serum were prepared as described in Example 2. Samples were prepared for 1:20, 1:40, 1:50, 1:60, 1:70 and 1:80 dilutions of normal serum. Test results are summarized in Figure 7. Normal serum samples exhibited no significant effect in comparison with BSK medium.

In addition, five pools (each containing 6 normal sera) were also examined. These pools of normal sera failed to reduce the number of organisms in the assay of the present invention when compared with BSK medium containing organisms.

### EXAMPLE 9

Normal and B. burgdorferi-infected sera were obtained from male LSH/SsLak hamsters (animals from Charles River Breeding Laboratories, Wilmington, MA). The animals were housed four per cage at ambient temperature. Normal serum was obtained from 6-8 week old hamsters.

B. burgdorferi sensitized (infected) serum was obtained from hamsters that had been injected with 0.2-0.4ml of 5 x 10⁶ cells/ml suspension of B. burgdorferi (strain 297). Injections were subcutaneous in the hind paws of the hamsters. Blood was drawn from infected hamsters by cardiac puncture at 3, 5 and 7 weeks after injection. Serum was obtained from the collected blood by standard techniques. The sera were filter sterilized, diluted 1:5 with BSK medium and tested in the assay of the present invention. Prior to use in the assays described below, the sera were heat inactivated for 45 minutes at 56C.

Sample mixtures were prepared with normal human serum, normal hamster serum and with 3, 5, and 7 week post-infection serum as described in Example 3. The samples were then incubated for 18 hours at 32C.

Triplicate samples of 10ul were removed from each incubated sample mixture and control mixture and counted as previously described. The sensitized serum samples exhibited significant decreases in the number of viable organisms in comparison to both the human and hamster normal serum controls. No significant difference was observed between the human and hamster normal serum controls.

Fresh BSK was added to samples which were then reincubated and counted as described in Example 4. Again, the counts confirmed that the number of viable organisms had actually been reduced.

### EXAMPLE 10

Sensitized serum samples from dogs have also been observed to significantly reduce the number of viable organisms remaining after treatment in accordance with the methods of the present invention. Normal serum from dogs did not reduce the number or organisms observed. Activity was also found in other animals (i.e., horses) suspected of having Lyme disease.

### EXAMPLE 11

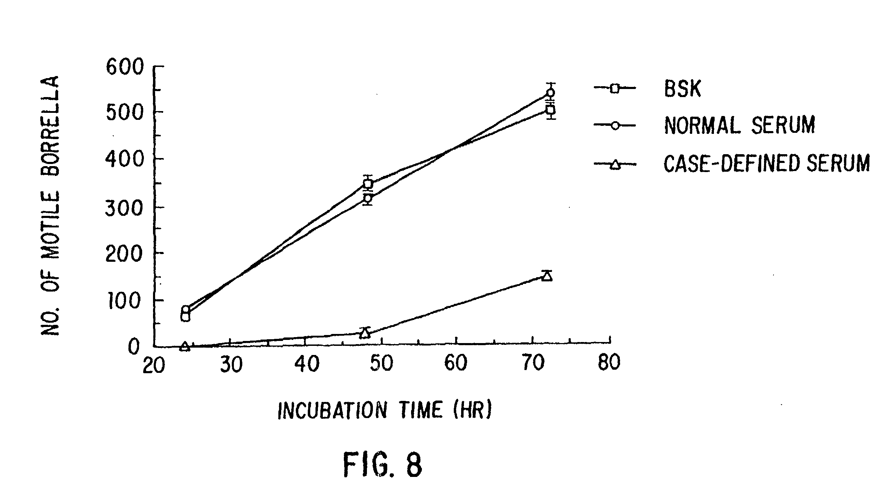

A normal serum and a Lyme disease serum were assayed as described in Example 1. After 18 hours of incubation, the Lyme disease serum caused a 97% reduction in motile B. burgdorferi compared to normal serum and BSK (not shown). Fresh BSK was added to samples which were then reincubated and counted as described in Example 4. After 24, 48, and 72 hours, the counts confirmed that the numbers of viable organisms had been reduced (Fig. 8).

### EXAMPLE 12

A radiolabel assay which utilizes the incorporation of ³H-adenine into viable B. burgdorferi was developed to determine the number of viable B. burgdorferi in sample and control mixtures. This increased the overall sensitivity of the assay by enabling fewer organisms to be used and decreasing the standard error between duplicate or triplicate samples. The assay was performed essentially as stated in Example 1. However, after the initial incubation, 2ul of ³H-adenine and 798ul of fresh BSK was added to the assay tubes. All assays were subsequently incubated for 4-6 additional days at 32C and the counts per minute of incorporated ³H-adenine was determined using scintillation counting.

### EXAMPLE 13

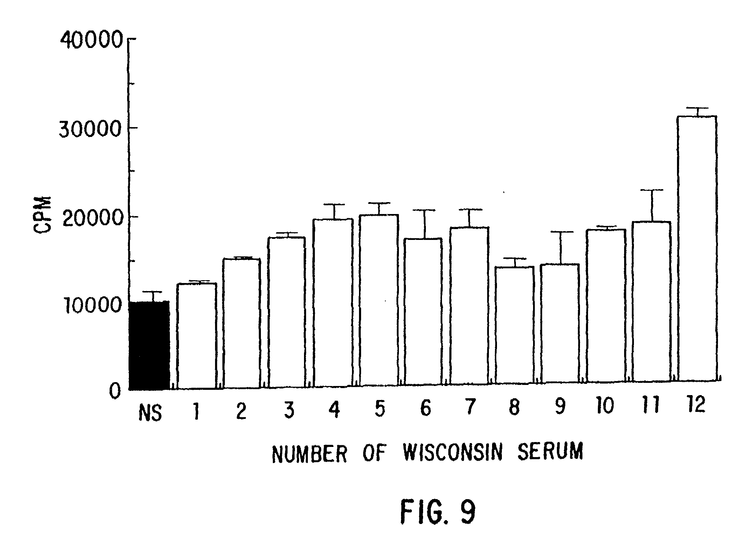

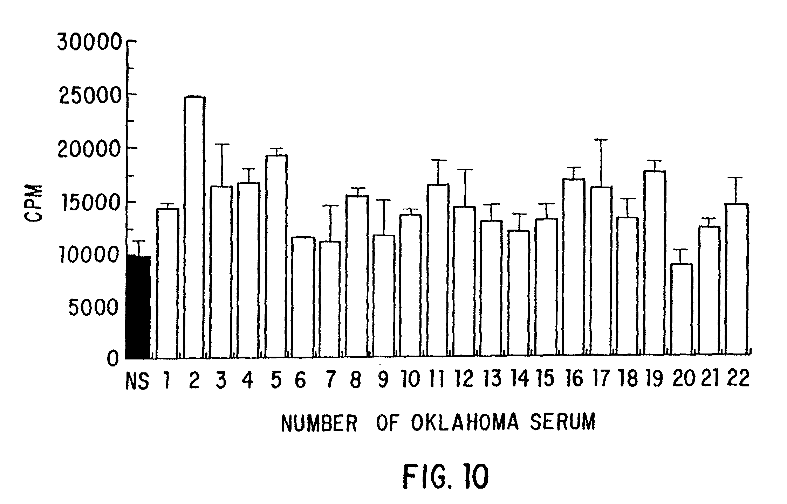

12 individual normal sera from Wisconsin and 22 individual normal sera from Oklahoma, which had previously been determined by case review and IFA to be negative for B. burgdorferi infection were analyzed in accordance with the procedure described in Example 12. Fig. 9 and 10 summarizes the borreliacidal activity. None of the tested normal serum caused B. burgdorferi killing compared to pooled normal sera.

### EXAMPLE 14

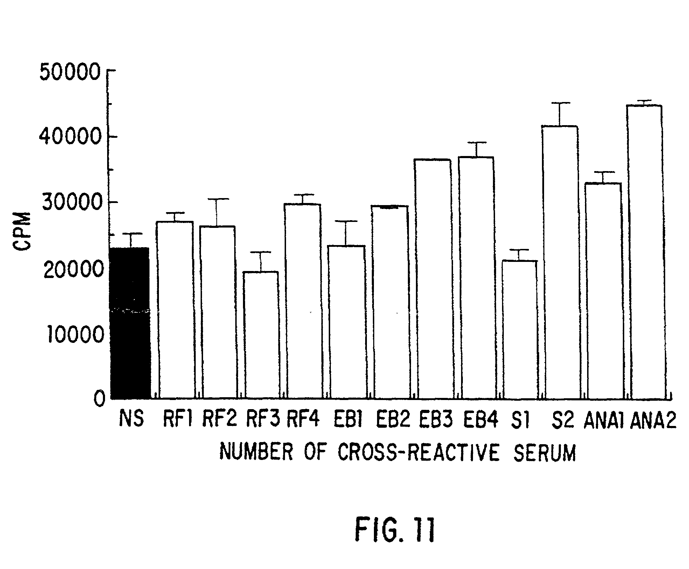

4 individual sera from patients with rheumatoid factor, 4 patients with mononucleosis, 2 patients with syphilis, and 2 patients with anti-nuclear antibody were analyzed in accordance with the procedure described in Example 12. Fig. 11 summarizes the borreliacidal activity. None of the potentially cross-reactive sera caused reductions of B. burgdorferi compared to pooled normal serum.

### EXAMPLE 15

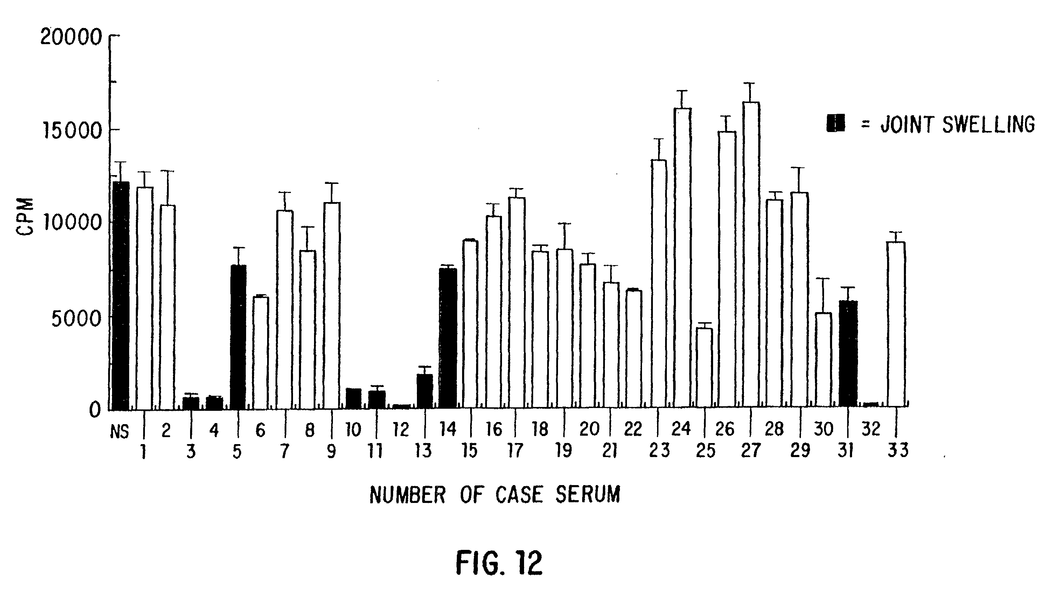

33 individual serum from patients diagnosed by a physician as having symptoms compatible with Lyme disease were analyzed as described in Example 12. Figure 12 summarizes the borreliacidal activity. 24 of 33 Lyme disease patients killed at least 10% of the added B. burgdorferi compared to pooled normal serum. When the patients who did not meet the strictest Center for Disease Control definition of Lyme disease were omitted, 20 of 20 case-defined sera caused reductions from 7 to 100%. In addition, 10 of 10 (100%) patients with joint swellings killed 38 to 100% of the B. burgdorferi.

### EXAMPLE 16

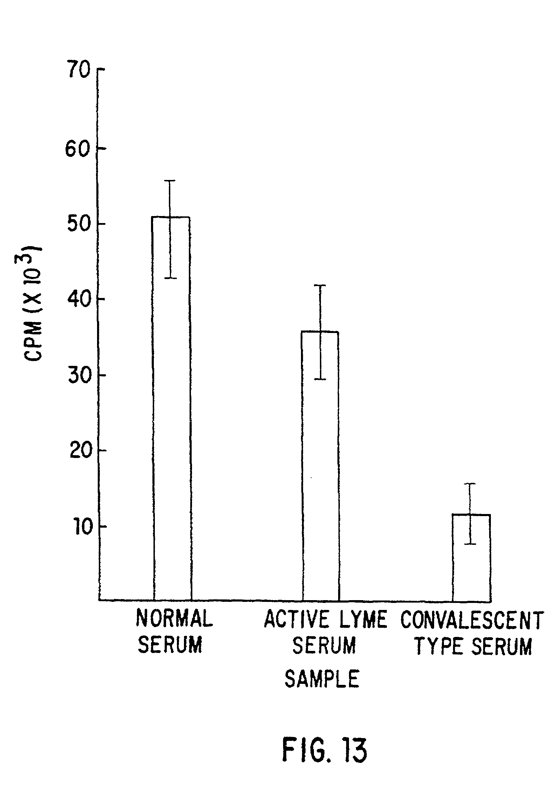

An acute and convalescent serum from a Lyme disease patient was analyzed as described in Example 12. Fig. 13 demonstrates that borreliacidal activity increased over time after infection with B. burgdorferi.

### EXAMPLE 17

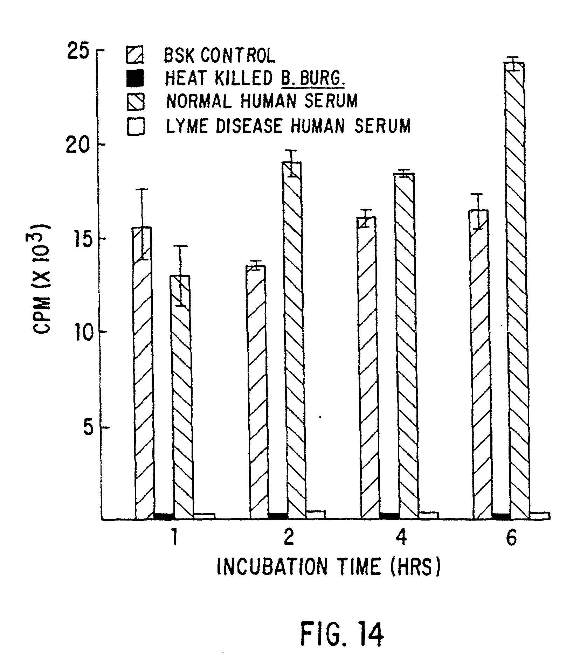

A Lyme disease serum and a normal human serum were assayed as described in Example 12 to determine how fast in vitro borreliacidal activity occurred. Identical samples were incubated for 1, 2, 4 or 6 hours at 32C before adding ³H-adenine and BSK. Figure 14 demonstrates that borreliacidal activity occurred as early as 1 hour after exposure of B. burgdorferi to Lyme disease serum.

### EXAMPLE 18

A counting assay which physically (mechanically) counts single bacteria was developed to increase the sensitivity and reproducibility of the assay and to eliminate the need for radioactive labels. The assay was performed essentially as stated in Example 1 and 12 except complement was added so that agglutination would not occur and individual organisms could be counted. After an initial incubation of 2 hours, 800ul of fresh BSK was added and the assays were incubated for 1-4 days to allow remaining organisms to multiply. After this incubation, the number of B. burgdorferi in aliquots of the individual samples was determined using a Coulter counter equipped with a 30 micron aperture.

### EXAMPLE 19

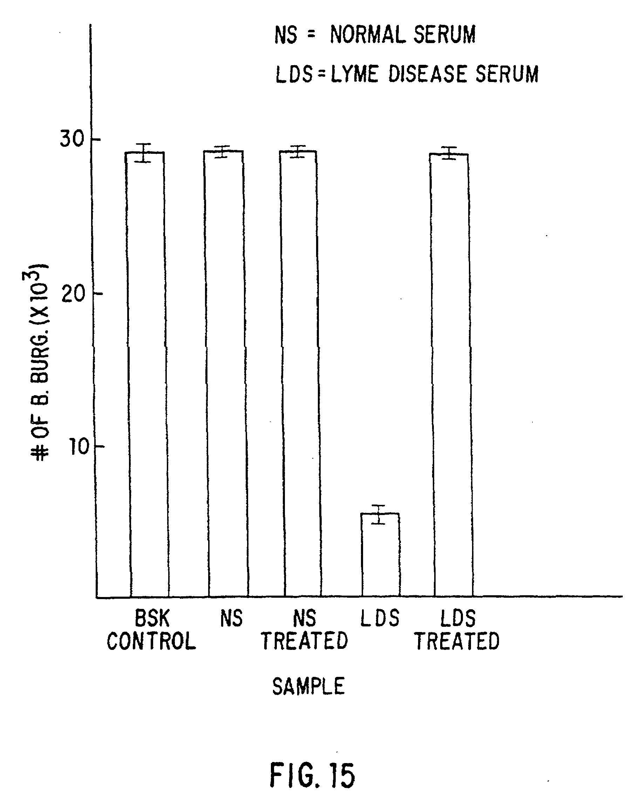

A normal serum and a Lyme disease serum were assayed for borreliacidal activity before and after treatment with human anti-IgG as described in Example 18. Fig. 15 demonstrates that treatment with anti-IgG did not affect normal serum. However, the removal of antibodies with anti-human IgG caused human Lyme disease serum to lose its borreliacidal activity. These results demonstrate that specific antibody is responsible for the borreliacidal activity in human Lyme disease serum.

### EXAMPLE 20

We have found that we can use labeling of B. burgdorferi with propidium iodide (15 ug/ml for 30 minute incubation) and flow cytometry to detect killed B. burgdorferi within 2 hours after the Lyme spirochete is added to case-defined serum with complement. Flow cytometry is used to count individual labelled bacteria. This method can determine live and dead bacteria by distinguishing between labelled and unlabelled bacteria.

### EXAMPLE 21

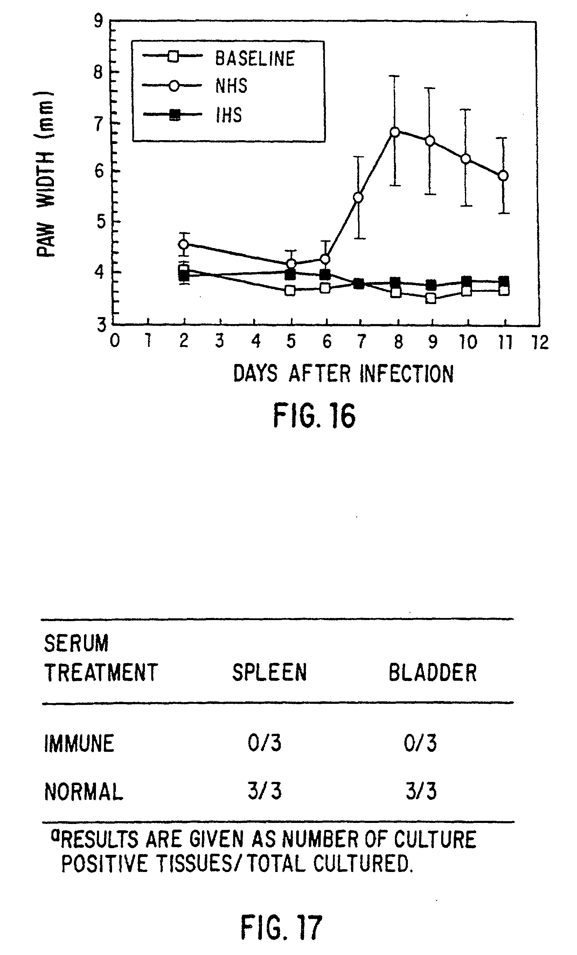

A case-defined Lyme disease serum was selected that demonstrated borreliacidal activity using our in vitro assay. The heat-inactivated serum was then injected intraperitoneally (0.5 ml) into 3 hamsters that were subsequently challenged with B. burgdorferi. Normal human serum (non-case) was included as a control. Figure 16 demonstrates that the human Lyme disease serum (B. burgdorferi positive serum) provided passive immunity and prevented hamsters from developing Lyme arthritis. More importantly, the case-defined Lyme serum killed B. burgdorferi in the hamsters (Figure 17). No spirochetes were recovered from the tissues of the hamsters. In contrast, spirochetes were recovered from the tissues of hamsters given normal (non-case) serum. These results also demonstrate that the in vitro assay of the present invention is a reliable predictor of immune status.

From the foregoing, it will be apparent to those skilled in the art that various modifications in the above-described methods, compositions, and articles of manufacture can be made without departing from the spirit and scope of the present invention. Accordingly, the present invention may be embodied in other specific forms without departing from the spirit or essential characteristics thereof. Present embodiments and examples, therefore, are to be considered in all respects as illustrative and not restrictive, the scope of the present invention being indicated by the appended claims rather than by the foregoing, and all changes which come within the meaning and range of equivalency of the claims are therefore intended to be embraced therein.

## Claims

1. An *in vitro* method of performing an assay to determine whether a patient has been exposed to Borrelia burgdorferi, said method comprising:
collecting serum from said patient;
preparing a sample mixture, said sample mixture comprising a portion of said patient's serum and an inoculum of viable Borrelia burgdorferi organisms;
incubating said sample mixture;
determining the number of viable organisms remaining in said sample mixture after incubation; and
comparing said number with the quantity of viable organisms remaining in a control.

2. A method according to Claim 1, wherein said serum is heat inactivated before preparation of said sample mixture.

3. A method according Claim 1, wherein said Borrelia burgdorferi organisms are of a strain selected from the group consisting of strain 297(ATCC 53899) strain B31 (ATCC 35210) and a strain indigenous to the area in which said patient is suspected to have been exposed to Borrelia burgdorferi.

4. A method according to Claim 3, wherein said Borrelia burgdorferi. organisms are of strain 297 (ATCC 53899).

5. A method according to Claim 3, wherein said Borrelia burgdorferi organisms are of a strain indigenous to the area in which said patient is suspected to have been exposed to Borrelia burgdorferi.

6. A method according to Claim 1, wherein said Borrelia burgdorferi organisms are properly aged organisms.

7. A method according to Claim 1, wherein said sample mixture and said control mixture are incubated at 32 °C.

8. A method accordingly to Claim 5, wherein said sample mixture is incubated for at least about 30 minutes.

9. A method accordingly to Claim 8, wherein said sample mixture is incubated for about 6 hours.

10. A method accordingly to Claim 8, wherein said sample mixture is incubated for about 18 hours.

11. A method according to Claim 1, wherein said number of viable organisms remaining in said sample mixture and said quantity of viable organisms remaining in said control mixture are determined by counting viable Borrelia burgdorferi organisms under a microscope.

12. A method according to Claim 1, wherein said patient is human.

13. A method according to Claim 1, wherein said control is prepared by forming a control mixture or normal serum and an inoculum of viable Borrelia burgdorferi organisms and said control mixture is incubated along with said sample mixture.

14. An assay kit useful for determining whether a patient has been exposed to or infected by Borrelia burgdorferi, said kit comprising:
an inoculum of viable Borrelia burgdorferi organisms, an inoculum of normal serum, and an aliquot of BSK medium.

15. An assay kit according to Claim 14, wherein said kit further comprises an aliquot of complement.

16. An assay kit according to Claim 14, wherein said Borrelia burgdorferi organisms are of a strain selected from the group consisting of strain 297 (ATCC 53899), strain B31 (ATCC 35210) and a strain indigenous to the area in which said patent is suspected to have been exposed to Borrelia burgdorferi.

17. An assay kit according to Claim 16, wherein said Borrelia burgdorferi organisms are of strain 297 (ATCC 53899).

18. An assay kit according to Claim 16, wherein said Borrelia burgdorferi organisms are of a strain indigenous to the area in which said patient is suspected to have been exposed to Borrelia burgdorferi.

19. An assay kit according to Claim 16, wherein said Borrelia burgdorferi organisms are properly aged organisms.

20. A method according to Claim 1, wherein said number of viable organisms remaining in said sample mixture and said quantity of viable organisms remaining in said control mixture are determined by measurement of uptake of ³H-adenine.

21. A method according to Claim 1, wherein said number of viable organisms remaining in said sample mixture and said quantity of viable organisms remaining in said control mixture are determined by flow cytometry or Coulter counter.

22. An assay kit according to Claim 14, wherein said kit further comprises an aliquot of a labelled or labelling material useful for counting viable Borrelia burgdorferi.

23. An assay kit according to Claim 22, wherein said labelling or labelling material is ³H-adenine.

24. An assay kit according to Claim 23, wherein said labeled or labeling material is propidium iodide.

## Patentansprüche

1. In-vitro-Verfahren zur Durchführung eines Tests um zu bestimmen, ob ein Patient *Borrelia burgdorferi* ausgesetzt wurde, wobei das Verfahren umfaßt:
Sammeln von Serum von dem Patienten;
Herstellen eines Probengemischs, wobei das Probengemisch einen Teil des Patientenserums und ein Inokulum von lebensfähigen *Borrelia-burgdorferi*-Organismen umfaßt;
Inkubieren des Probengemischs;
Bestimmen der Zahl von lebensfähigen Organismen, die in der Probe nach der Inkubation verbleiben; und
Vergleichen der Zahl mit der Menge von lebensfähigen Organismen, die in einer Kontrolle verbleiben.

2. Verfahren nach Anspruch 1, wobei das Serum vor der Herstellung des Probengemischs Hitze-inaktiviert wird.

3. Verfahren nach Anspruch 1, worin die *Borrelia burgdorferi-*Organismen von einem Stamm ausgewählt werden aus der Gruppe bestehend aus Stamm 297 (ATTC 53899), Stamm B31 (ATTC 35210) und einem Stamm, der aus der Region stammt, von der vermutet wird, daß der Patient dort *Borrelia burgdorferi* ausgesetzt wurde.

4. Verfahren nach Anspruch 3, wobei die *Borrelia burgdorferi*-Organismen vom Stamm 297 (ATTC 53899) sind.

5. Verfahren nach Anspruch 3, wobei die *Borrelia burgdorferi*-Organismen von einem Stamm sind, der aus der Region stammt, von der vermutet wird, daß der Patient dort *Borrelia burgdorferi* ausgesetzt wurde.

6. Verfahren nach Anspruch 1, wobei die *Borrelia burgdorferi-*Organismen geeignet gealterte Organismen sind.

7. Verfahren nach Anspruch 1, wobei das Probengemisch und das Kontrollgemisch bei 32° C inkubiert werden.

8. Verfahren nach Anspruch 5, wobei das Probengemisch für mindestens ungefähr 30 Minuten inkubiert wird.

9. Verfahren nach Anspruch 8, wobei das Probengemisch für ungefähr 6 Stunden inkubiert wird.

10. Verfahren nach Anspruch 8, wobei das Probengemisch für ungefähr 18 Stunden inkubiert wird.

11. Verfahren nach Anspruch 1, wobei die Zahl von lebensfähigen Organismen, die in dem Probengemisch verbleiben, und die Menge von lebensfähigen Organismen, die in dem Kontrollgemisch verbleiben, durch Zählen von lebensfähigen *Borrelia burgdorferi*-Organismen unter einem Mikroskop bestimmt werden.

12. Verfahren nach Anspruch 1, wobei der Patient menschlich ist.

13. Verfahren nach Anspruch 1, wobei die Kontrolle durch Bilden eines Kontrollgemischs oder normalem Serum und einem Inokulum von lebensfähigen *Borrelia burgdorferi*-Organismen hergestellt wird, und das Kontrollgemisch zusammen mit dem Probengemisch inkubiert wird.

14. Testkit, brauchbar um zu bestimmen, ob ein Patient gegenüber *Borrelia burgdorferi* exponiert oder durch *Borrelia burgdorferi* infiziert wurde, wobei das Kit umfaßt:
Ein Inokulum von lebensfähigen *Borrelia burgdorferi*-Organismen, ein Inokulum von normalem Serum und einen Aliquot BSK-Medium.

15. Testkit nach Anspruch 14, wobei das Kit weiterhin einen Aliquot von Komplement umfaßt.

16. Testkit nach Anspruch 14, wobei die *Borrelia burgdorferi*-Organismen von einem Stamm ausgewählt aus der Gruppe bestehend aus Stamm 297 (ATTC 53899), Stamm B31 (ATTC 35210) und einem Stamm sind, der aus der Region stammt, von der vermutet wird, daß der Patient dort *Borrelia burgdorferi* ausgesetzt wurde.

17. Testkit nach Anspruch 16, wobei die *Borrelia burgdorferi-Organismen* vom Stamm 297 (ATTC 53899) sind.

18. Testkit nach Anspruch 16, wobei die *Borrelia burgdorferi-*Organismen von dem Stamm sind, der aus der Region stammt, von der vermutet wird, daß der Patient dort *Borrelia burgdorferi* ausgesetzt wurde.

19. Testkit nach Anspruch 16, wobei die *Borrelia burgdorferi*-Organismen geeignet gealterte Organismen sind.

20. Verfahren nach Anspruch 1, wobei die Zahl von lebensfähigen Organismen, die in dem Probengemisch verbleiben, und die Menge der lebensfähigen Organismen, die in dem Kontrollgemisch verbleiben, durch Messen der Aufnahme von ³H-Adenin bestimmt wird.

21. Verfahren nach Anspruch 1, wobei die Zahl von lebensfähigen Organismen, die in dem Probengemisch verbleiben, und die Menge von lebensfähigen Organismen, die in dem Kontrollgemisch verbleiben, durch Flußzytometrie oder ein Coulter-Zählgerät bestimmt wird.

22. Testkit nach Anspruch 14, wobei das Kit weiterhin ein Aliquot von markiertem oder markierendem Material umfaßt, das zum Zählen von lebensfähigen *Borrelia burgdorferi* brauchbar ist.

23. Testkit nach Anspruch 22, wobei das markierte oder markierende Material ³H-Adenin ist.

24. Testkit nach Anspruch 23, wobei das markierte oder markierende Material Propidiumjodid ist.

## Revendications

1. Procédé in vitro pour réaliser un test pour déterminer si un patient a été exposé à Borrelia burgdorferi, ledit procédé comprenant :
- recueillir du sérum à partir dudit patient ;
- préparer un mélange échantillon, ledit mélange échantillon comprenant une portion dudit sérum du patient et un inoculum d'organismes viables Borrelia burgdorferi ;
- incuber ledit mélange échantillon ;
- déterminer le nombre d'organismes viables demeurant dans ledit mélange échantillon après incubation ; et
- comparer ledit nombre avec la quantité d'organismes viables demeurant dans un témoin.

2. Procédé selon la revendication 1, dans lequel ledit sérum est inactivé à la chaleur avant la préparation dudit mélange échantillon.

3. Procédé selon la revendication 1, dans lequel lesdits organismes Borrelia burgdorferi sont d'une souche choisie dans le groupe constitué de la souche 297(ATCC 53899), la souche B31 (ATCC 35210) et une souche indigène à la région dans laquelle ledit patient est suspecté d'avoir été exposé à Borrelia burgdorferi.

4. Procédé selon la revendication 3, dans lequel lesdits organismes Borrelia burgdorferi sont de souche 297(ATCC 53899).

5. Procédé selon la revendication 3, dans lequel lesdits organismes Borrelia burgdorferi sont d'une souche indigène à la région dans laquelle ledit patient est suspecté d'avoir été exposé à Borrelia burgdorferi.

6. Procédé selon la revendication 1, dans lequel lesdits organismes Borrelia burgdorferi sont des organismes d'âge approprié.

7. Procédé selon la revendication 1, dans lequel ledit mélange échantillon et ledit mélange témoin sont incubés à 32°C.

8. Procédé selon la revendication 5, dans lequel ledit mélange échantillon est incubé pendant au moins environ trente minutes.

9. Procédé selon la revendication 8, dans lequel ledit mélange échantillon est incubé pendant environ six heures.

10. Procédé selon la revendication 8, dans lequel ledit mélange échantillon est incubé pendant environ dix-huit heures.

11. Procédé selon la revendication 1, dans lequel ledit nombre d'organismes viables demeurant dans ledit mélange échantillon et ladite quantité d'organismes viables demeurant dans ledit mélange témoin sont déterminés en comptant les organismes Borrelia burgdorferi viables sous un microscope.

12. Procédé selon la revendication 1, dans lequel ledit patient est humain.

13. Procédé selon la revendication 1, dans lequel ledit témoin est préparé en formant un mélange témoin de sérum normal et d'un inoculum d'organismes Borrelia burgdorferi viables et ledit mélange témoin est incubé en même temps que ledit mélange échantillon.

14. Kit de test utile pour déterminer si un patient a été exposé à ou infecté par Borrelia burgdorferi, ledit kit comprenant un inoculum d'organismes Borrelia burgdorferi viables, un inoculum de sérum normal, et une aliquote de milieu BSK.

15. Kit de test selon la revendication 14, dans lequel ledit kit comprend de plus une aliquote de complément.

16. Kit de test selon la revendication 14, dans lequel lesdits organismes Borrelia burgdorferi sont d'une souche choisie dans le groupe constitué de la souche 297(ATCC 53899), la souche B31 (ATCC 35210) et une souche indigène à la région dans laquelle ledit patient est suspecté d'avoir été exposé à Borrelia burgdorferi.

17. Kit de test selon la revendication 16, dans lequel lesdits organismes Borrelia burgdorferi sont de souche 297(ATCC 53899).

18. Kit de test selon la revendication 16, dans lequel lesdits organismes Borrelia burgdorferi sont d'une souche indigène à la région dans laquelle ledit patient est suspecté d'avoir été exposé à Borrelia burgdorferi.

19. Kit de test selon la revendication 16, dans lequel lesdits organismes Borrelia burgdorferi sont des organismes d'âge approprié.

20. Procédé selon la revendication 1, dans lequel ledit nombre d'organismes viables demeurant dans ledit mélange échantillon et ladite quantité d'organismes viables demeurant dans ledit mélange témoin sont déterminés par mesure d'absorption de ³H-adénine.

21. Procédé selon la revendication 1, dans lequel ledit nombre d'organismes viables demeurant dans ledit mélange échantillon et ladite quantité d'organismes viables demeurant dans ledit mélange témoin sont déterminés par cytométrie à flux ou par compteur Coulter.

22. Kit de test selon la revendication 14, dans lequel ledit kit comprend de plus une aliquote d'une matière marquée ou marquante utile pour compter les Borrelia burgdorferi viables.

23. Kit de test selon la revendication 22, dans lequel ladite matière marquée ou marquante est ³H-adénine.

24. Kit de test selon la revendication 23, dans lequel ladite matière marquée ou marquante est de l'iodure de propidium.
